Europäisches Patentamt

European Patent Office

Office européen des brevets

(11) Numéro de publication: **0 449 722 B1**

## FASCICULE DE BREVET EUROPEEN

(45) Date de publication de fascicule du brevet: **19.10.94**  (51) Int. Cl.5: **C07D 333/24**, C08B 37/16, A61K 31/38, A61K 31/715

(21) Numéro de dépôt: **91400809.9**

(22) Date de dépôt: **26.03.91**

(54) **Nouveaux complexes de l'acide tiaprofénique ou de ses esters insolubles ou partiellement solubles avec les cyclodextrines ou leurs dérivés.**

(30) Priorité: **27.03.90 FR 9003891**

(43) Date de publication de la demande:
**02.10.91 Bulletin 91/40**

(45) Mention de la délivrance du brevet:
**19.10.94 Bulletin 94/42**

(84) Etats contractants désignés:
**CH DE FR GB IT LI NL**

(56) Documents cités:
**EP-A- 0 268 215**
**WO-A-85/02767**
**FR-A- 2 319 340**

**CHEMICAL ABSTRACTS, vol. 103, no. 20, 18 novembre 1985, page 359, résumé no.166008d, Columbus, Ohio, US; T. WAKUDA et al.: "Prostaglandins and theircyclodextrin complexes", & J. INCLUSION PHENOM. 1984,2(3-4), 467-74**

(73) Titulaire: **ROUSSEL-UCLAF**
**35, Boulevard des Invalides**
**F-75007 Paris (FR)**

(72) Inventeur: **Cohen, Gérard**
**7, Avenue Daniel Lesueur**
**F-75007 Paris (FR)**
Inventeur: **Dubois, Jean-Luc**
**63, rue de Meaux**
**F-75019 Paris (FR)**

(74) Mandataire: **Jacobi, Markus Alexander et al**
**Roussel Uclaf**
**111, Route de Noisy**
**B.P. 9**
**F-93230 Romainville (FR)**

## Description

La présente invention concerne de nouveaux complexes de l'acide tiaprofénique ou de ses esters, avec les cyclodextrines ou leurs dérivés, leur procédé de préparation, leurs applications à titre de médicament et les compositions les renfermant.

En raison du caractère peu soluble de l'acide tiaprofénique dans l'eau, la demanderesse a été amenée à rechercher un nouveau complexe d'inclusion de cet acide ou de ses esters, au sein de molécules plus solubles, les cyclodextrines, afin d'augmenter la solubilité de l'acide tiaprofénique, son absorption in vivo et donc sa biodisponibilité.

De plus ce complexe présente les avantages de protéger la muqueuse gastrique, de diminuer les posologies usuelles, de masquer le gout et l'amertume du principe actif permettant ainsi une meilleure utilisation des sachets, comprimés, gélules destinés à être administrés par voie orale.

L'augmentation de la solubilité de l'acide tiaprofénique permet aussi son utilisation, à titre de médicament, destiné notamment à la voie parentérale.

L'acide tiaprofénique est décrit dans le brevet français n° 2068425 et son sel de tromethamine dans le brevet français n° 2319340, tous deux déposés par la demanderesse. Les cyclodextrines et leurs dérivés sont décrits dans la demande de brevet internationale PCT/EP 84/00417.

La présente invention a ainsi pour objet les nouveaux complexes de l'acide tiaprofénique ou de ses esters insolubles ou partiellement solubles avec les cyclodextrines ou leurs dérivés, caractérisés en ce que les cyclodextrines sont représentées par l'alpha-cyclodextrine, la béta-cyclodextrine ou la gamma-cyclodextrine.

La présente invention a aussi pour objet les nouveaux complexes de l'acide tiaprofénique ou de ses esters insolubles ou partiellement solubles avec les cyclodextrines ou leurs dérivés, caractérisés en ce que l'on utilise comme dérivés de la cyclodextrine soit une alkyl béta-cyclodextrine soit une hydroxy alkyl béta-cyclodextrine.

Dans l'expression alkyl béta-cyclodextrine, le terme alkyl désigne un radical alkyle renfermant de 1 à 5 atomes de carbone et de préférence un radical méthyle, éthyle, propyle ou isopropyle. Parmi les produits qui constituent l'objet de l'invention, on peut citer notamment la méthyl bétacyclodextrine ou l'éthyl béta-cyclodextrine.

Dans l'expression hydroxy alkyl béta-cyclodextrine, le terme hydroxy alkyl désigne un radical hydroxy alkyle renfermant de 1 à 5 atomes de carbone et de 1 à 2 groupements hydroxyles, tel que l'hydroxy éthyle, l'hydroxy propyle, le dihydroxy propyle. Parmi les produits qui constituent l'objet de l'invention, on peut citer notamment l'hydroxy propyl bétacyclodextrine.

La présente invention a ainsi pour objet plus particulièrement le nouveau complexe de l'acide tiaprofénique et de l'hydroxy propyl béta-cyclodextrine.

La présente invention concerne aussi un procédé de préparation des nouveaux complexes de l'acide tiaprofénique ou de ses esters insolubles ou partiellement solubles avec les cyclodextrines ou leurs dérivés, procédé caractérisé en ce que l'on mélange l'acide tiaprofénique ou l'un de ses esters avec le dérivé de la cyclodextrine, puis isole le complexe ainsi formé.

Le mélange de l'acide tiaprofénique ou de l'un de ses esters avec le dérivé de la cyclodextrine peut s'effectuer au sein d'un solvant aqueux acide ou alcalin, ou organique tel que l'éthanol, le méthanol, l'acétone, l'éther éthylique.

Dans les conditions préférentielles de mise en oeuvre de l'invention, on dissout sous agitation continue la cyclodextrine ou l'un de ses dérivés dans de l'eau dont la température varie de 15 à 45°C, et plus particulièrement à 37°C ; la solution devient limpide en quelques minutes. Le principe actif est ajouté ensuite, entrainant la formation d'un trouble qui disparait au bout d'une vingtaine de minutes. On laisse la solution au repos à température ambiante quelques heures, puis on récupère le précipité qui s'est formé, le lave, et le sèche. On peut améliorer le rendement en abaissant la température de la solution pendant la précipitation.

Les nouveaux complexes de l'acide tiaprofénique ou de ses esters insolubles ou partiellement solubles avec les cyclodextrines ou leurs dérivés, qui constituent l'objet de la présente demande, conservent les propriétés pharmacologiques de l'acide tiaprofénique, à savoir de remarquables propriétés antalgiques, anti-inflammatoires et antipyrétiques.

Les nouveaux complexes tel qu'obtenus dans la mise en oeuvre du procédé précité présentent l'avantage d'avoir une solubilité dans l'eau supérieure à celle rapportée dans la littérature concernant le sel de tiaprofénique et de l'hydroxy propyl béta-cyclodextrine présente une solubilité dans l'eau supérieure à 500 mg/ml.

Le nouveau complexe de l'acide tiaprofénique et de l'hydroxy propyl béta-cyclodextrine présente une solubilité dans l'eau supérieure à 500 mg/ml.

Ces propriétés physiques, alliées aux remarquables propriétés pharmacologiques justifient l'utilisation des nouveaux complexes de l'acide tiaprofénique ou de ses esters insolubles ou partiellement solubles avec les cyclodextrines ou leurs dérivés à titre de médicament.

La présente demande a ainsi également pour objet l'application à titre de médicament des nouveaux complexes de l'acide tiaprofénique ou de ses esters insolubles ou partiellement solubles avec les cyclodextrines ou leurs dérivés. Leurs propriétés pharmacologiques les rendent aptes à être utilisés dans le traitement des algies musculaires, articulaires ou nerveuses, des affections rhumatismales, des migraines, des douleurs dentaires.

La dose usuelle peut varier par exemple de 50 à 300 mg par jour selon la voie d'administration, le sujet traité et l'affection en cause.

L'invention a encore pour objet les compositions pharmaceutiques qui renferment au moins le médicament précité à titre de principe actif.

L'invention a enfin pour objet les compositions pharmaceutiques qui contiennent l'acide tiaprofénique ou ses esters insolubles ou partiellement solubles, et les cyclodextrines ou leurs dérivés, ces 2 agents étant séparés physiquement pour une utilisation simultanée, séparée ou étalée dans le temps.

Les compositions qui constituent l'objet de la présente demande peuvent être réalisées de façon à pouvoir être administrées par la voie orale ou parentérale. Elles sont préparées selon les méthodes usuelles. Le ou les principes actifs peuvent y être incorporés à des excipients habituellement employés dans ces compositions pharmaceutiques tels que le talc, le lactose, le mannitol, l'amidon, le stéarate de magnésium, les véhicules aqueux ou non, les conservateurs.

On utilise de préférence l'hydroxy propyl bétacyclodextrine pour les préparations injectables.

Il va être donné maintenant à titre non limitatif des exemples de mise en oeuvre de l'invention.

**Exemple 1 : Préparation du complexe de l'acide tiaprofénique avec la béta-cyclodextrine.**

17 g de béta-cyclodextrine sont dissous sous agitation continue dans de l'eau à 37°C qsp 1 litre, la solution devient limpide en quelques minutes. 2,6 g d'acide tiaprofénique sont ajoutés ensuite à cette solution, entrainant la formation d'un trouble qui disparait en une vingtaine de minutes. La solution est laissée à température ambiante quelques heures ; il apparait alors un précipité qui est récupéré, lavé, séché, et qui représente environ 10 grammes de complexe à 17% en poids d'acide tiaprofénique. On augmente le rendement en diminuant la température de la solution pendant la précipitation.

**Exemple 2 : Préparation d'une solution à base du complexe acide tiaprofénique, hydroxy propyl béta-cyclodextrine, renfermant 10 mg/ml d'acide tiaprofénique.**

On prépare 50 ml de solution A d'hydroxy propyl bétacyclodextrine à 150 mg/ml en dissolvant sous agitation continue 7,5 g d'hydroxy propyl béta-cyclodextrine dans de l'eau distillée à température ambiante qsp 50 ml. Puis on ajoute, à la solution A, 500 mg d'acide tiaprofénique dont la dissolution à température ambiante sera complète en 30 minutes. On laisse sous agitation 1 heure puis on filtre stérilement sur membrane, et on récupère la solution.

**Exemple 3 : On a préparé des sachets répondant à la formule suivante.**

| | |
|---|---|
| Acide tiaprofénique | 52,5 mg |
| Béta-cyclodextrine | 750 mg |
| Excipient qsp 1 sachet terminé à | 2 g |
| (Composition de l'excipient : sucre glace, aerosil 200, arôme banane, arôme abricot). Surcharge de 5 % en principe actif pour stabilité. | |

**Exemple 4 : Soluté injectable à base du complexe acide tiaprofénique, hydroxy propyl béta-cyclodextrine.**

Présentation : un flacon de poudre stérile à dissoudre extemporanément dans un solvant approprié.

**Formule** :

I) Flacon de poudre stérile :

| - acide tiaprofénique lyophylise | 100 mg |
|---|---|
| - Mannitol | 50 mg |

II) Ampoule de solvant :

| - hydroxy propyl béta-cyclodextrine | 500 mg |
|---|---|
| - chlorure de sodium | 36 mg |
| - eau distillée qsp | 5 ml |

**Mode opératoire** :

I) L'acide tiaprofénique est microbroyé en présence de mannitol, le mélange est réparti en flacons stériles.

II) Solvant : l'hydroxy propyl béta-cyclodextrine et le chlorure de sodium sont dissous dans l'eau. La solution filtrée est répartie en ampoules que l'on stérilise par autoclavage.

Etude de la biodisponibilité de l'acide tiaprofénique sous la forme de complexe acide tiaprofénique béta-cyclodextrine.

Cette étude a été réalisée par comparaison vis à vis du comprimé standard d'acide tiaprofénique dosé à 100 mg, le complexe acide tiaprofénique béta-cyclodextrine étant utilisé sous forme de sachet dosé à 50 mg.

On a administré selon un mode croisé randomisé l'acide tiaprofénique à 12 volontaires sains, de sexe masculin, âgés de 18 à 40 ans et dont les examens cliniques et biologiques ont été considérés comme normaux par l'expérimentateur. Une période sans traitement d'une semaine a été respectée entre chaque cure. Des prélèvements de sang veineux ont été effectués au temps 0, juste avant l'administration et 0,25, 0,50, 0,75, 1, 1,5, 2, 3, 4, 6, 8, 10 et 12 heures après la prise du produit.

Pour chacune des 2 formes on a retenu les quatre paramètres suivants :

$C_{max}$ est la concentration plasmatique maximale observée au cours de la cinétique et est exprimée en mg par litre.

$T_{max}$ est le temps mis pour atteindre la concentration plasmatique maximale exprimé en heures.

AUC est l'aire sous la courbe calculée par la méthode des trapezes entre le temps zéro et le dernier temps où le produit est détectable, exprimée en mg par litre par heure.

MRT est le temps de séjour moyen de l'acide tiaprofénique dans l'organisme, calculé d'après les concentrations observées

$$MRT = \frac{AUMC}{AUC} \qquad AUMC = \int t.c.dt \quad et \quad AUC = \int c.dt$$

| | Comprimé | Sachet |
|---|---|---|
| $C_{max}$ | 12,09 ± 0,85 | 14,28 ± 0,79 |
| $T_{max}$ | 0,85 ± 0,11 | 0,479 ± 0,048 |
| AUC | 22,91 ± 0,72 | 22,82 ± 0,96 |
| MRT | 2,33 ± 0,17 | 1,940 ± 0,054 |

Les valeurs des concentrations plasmatiques déterminées aux temps 0,25, 0,5, 0,75 et 1 heure après la prise du produit sont regroupées dans le tableau suivant :

4

| Temps (h) | 0,25 | 0,50 | 0,75 | 1 |
|-----------|------|------|------|---|
| Comprimé | 3 ± 1 | 9,4 ± 1,4 | 10,2 ± 1,3 | 8,94 ± 0,87 |
| Sachet | 11 ± 1,4 | 13,3 ± 0,79 | 10,76 ± 0,5 | 8,40 ± 0,50 |

Ces valeurs et notamment la valeur de la concentration maximale, paramètre caractérisant les phénomènes d'absorption, significativement plus élevées avec la nouvelle forme complexe acide tiaprofénique béta-cyclodextrine, témoignent d'une absorption accrue, due à un passage membranaire facilité par une plus grande solubilité du principe actif.

## Revendications

1. Nouveaux complexes de l'acide tiaprofénique ou de ses esters insolubles ou partiellement solubles avec les cyclodextrines ou leurs dérivés.

2. Complexe selon la revendication 1 caractérisé en ce que les cyclodextrines peuvent être représentées par l'alpha-cyclodextrine, la béta-cyclodextrine ou la gamma-cyclodextrine.

3. Complexe selon la revendication 1 ou 2 caractérisé en ce que le dérivé de la cyclodextrine est une alkyl béta-cyclodextrine.

4. Complexe selon la revendication 3 caractérisé en ce que l'alkyl béta-cyclodextrine est représentée notamment par la méthyl béta-cyclodextrine ou par l'éthyl béta-cyclodextrine.

5. Complexe selon la revendication 1 ou 2 caractérisé en ce que le dérivé de la cyclodextrine est une hydroxy alkyl béta-cyclodextrine.

6. Complexe selon la revendication 5, caractérisé en ce que l'hydroxy alkyl béta-cyclodextrine est représentée notamment par l'hydroxy propyl béta-cyclodextrine.

7. Nouveau complexe constitué par l'acide tiaprofénique et l'hydroxy propyl béta-cyclodextrine.

8. Procédé de préparation des nouveaux complexes tels que définis à la revendication 1 caractérisé en ce que l'on mélange l'acide tiaprofénique ou l'un de ses esters avec les cyclodextrines ou leurs dérivés puis récupère le complexe ainsi formé.

9. Procédé de préparation selon la revendication 8, caractérisé en ce que le mélange de l'acide tiaprofénique ou de l'un de ses esters avec les cyclodextrines ou leurs dérivés s'effectue au sein d'un solvant.

10. Nouveau complexe tel qu'obtenu dans la mise en oeuvre du procédé défini à l'une quelconque des revendications 8 ou 9.

11. Médicament, caractérisé en ce qu'il est constitué par les nouveaux complexes tels que définis à la revendication 1.

12. Médicament, caractérisé en ce qu'il est constitué par les nouveaux complexes tels que définis à l'une quelconque des revendications 2, 3, 4, 5, 6, 7, 10.

13. Compositions pharmaceutiques, caractérisées en ce qu'elles renferment à titre de principe actif, l'un au moins des médicaments tels que définis à l'une quelconque des revendications 11 et 12.

14. Compositions pharmaceutiques, caractérisées en ce qu'elles contiennent l'acide tiaprofénique ou ses esters insolubles ou partiellement solubles, et les cyclodextrines ou leurs dérivés, ces 2 agents étant séparés physiquement pour une utilisation simultanée, séparée ou étalée dans le temps.

**Claims**

1. New complexes of tiaprofenic acid or its insoluble or partially soluble esters with cyclodextrins or their derivatives.

2. Complex according to claim 1 characterized in that the cyclodextrins can be represented by alpha-cyclodextrin, beta-cyclodextrin or gamma-cyclodextrin.

3. Complex according to claim 1 or 2 characterized in that the cyclodextrin derivative is an alkyl beta-cyclodextrin.

4. Complex according to claim 3 characterized in that the alkyl beta-cyclodextrin is represented in particular by methyl beta-cyclodextrin or by ethyl beta-cyclodextrin.

5. Complex according to claim 1 or 2 characterized in that the cyclodextrin derivative is a hydroxy alkyl beta-cyclodextrin.

6. Complex according to claim 5, characterized in that the hydroxy alkyl beta-cyclodextrin is represented in particular by hydroxy propyl beta-cyclodextrin.

7. New complex constituted by tiaprofenic acid and hydroxy propyl beta-cyclodextrin.

8. Preparation process for new complexes as defined in claim 1 characterized in that tiaprofenic acid or one of its esters is mixed with cyclodextrins or their derivatives then the complex thus formed is collected.

9. Preparation process according to claim 8, characterized in that the mixing of tiaprofenic acid or one of its esters with cyclodextrins or their derivatives is carried out in a solvent.

10. New complex as obtained in the implementation of the process defined in any one of claims 8 or 9.

11. Medicament, characterized in that it is constituted by the new complexes as defined in claim 1.

12. Medicament, characterized in that it is constituted by the new complexes as defined in any one of claims 2, 3, 4, 5, 6, 7, 10.

13. Pharmaceutical compositions, characterized in that they contain as active ingredient at least one of the medicaments as defined in any one of claims 11 and 12.

14. Pharmaceutical compositions, characterized in that they contain tiaprofenic acid or its insoluble or partially soluble esters, and cyclodextrins or their derivatives, these two agents being physically separate for a simultaneous or separate use or a use spread out over time.

**Patentansprüche**

1. Neue Komplexe der Tiaprofensäure oder ihrer unlöslichen oder teilweise löslichen Ester mit Cyclodextrinen oder deren Derivaten.

2. Komplex nach Anspruch 1, dadurch gekennzeichnet, daß die Cyclodextrine durch alpha-Cyclodextrin, beta-Cyclodextrin oder gamma-Cyclodextrin repräsentiert sein können.

3. Komplex nach Anspruch 1 oder 2, dadurch gekennzeichnet, daß das Derivat des Cyclodextrins ein Alkyl-beta-cyclodextrin ist.

4. Komplex nach Anspruch 3, dadurch gekennzeichnet, daß das Alkyl-beta-cyclodextrin insbesondere durch Methyl-beta-cyclodextrin oder durch Ethyl-beta-cyclodextrin repräsentiert wird.

5. Komplex nach Anspruch 1 oder 2, dadurch gekennzeichnet, daß das Derivat des Cyclodextrins ein Hydroxyalkyl-beta-cyclodextrin ist.

6. Komplex nach Anspruch 5, dadurch gekennzeichnet, daß das Hydroxyalkyl-beta-cyclodextrin insbesondere durch Hydroxypropyl-beta-cyclodextrin repräsentiert wird.

7. Neuer Komplex, bestehend aus Tiaprofensäure und Hydroxypropyl-beta-cyclodextrin.

8. Verfahren zur Herstellung der gemäß Anspruch 1 definierten neuen Komplexe, dadurch gekennzeichnet, daß man Tiaprofensäure oder einen ihrer Ester mit Cyclodextrinen oder deren Derivaten mischt, dann den so gebildeten Komplex gewinnt.

9. Verfahren zur Herstellung nach Anspruch 8, dadurch gekennzeichnet, daß die Mischung von Tiaprofensäure oder eines ihrer Ester mit den Cyclodextrinen oder deren Derivaten in einem Lösungsmittel bewirkt wird.

10. Neuer Komplex, wie durch die Durchführung des durch irgendeinen der Ansprüche 8 oder 9 definierten Verfahrens erhalten.

11. Medikament, dadurch gekennzeichnet, daß es aus neuen Komplexen, wie in Anspruch 1 definiert, besteht.

12. Medikament, dadurch gekennzeichnet, daß es aus neuen Komplexen, wie in irgendeinem der Ansprüche 2, 3, 4, 5, 6, 7, 10 definiert, besteht.

13. Pharmazeutische Zusammensetzungen, dadurch gekennzeichnet, daß sie als Wirkstoff mindestens ein Medikament, wie in irgendeinem der Ansprüche 11 und 12 definiert, umfassen.

14. Pharmazeutische Zusammensetzungen, dadurch gekennzeichnet, daß sie Tiaprofensäure oder ihre unlöslichen oder teilweise löslichen Ester und die Cyclodextrine oder deren Derivate enthalten, wobei diese zwei Mittel physikalisch getrennt für eine gleichzeitige, getrennte oder zeitlich gestaffelte Verwendung vorliegen.